(19) Europäisches Patentamt
European Patent Office
Office européen des brevets

(11) EP 0 504 673 B1

(12) EUROPÄISCHE PATENTSCHRIFT

(45) Veröffentlichungstag und Bekanntmachung des Hinweises auf die Patenterteilung:
12.06.1996 Patentblatt 1996/24

(51) Int Cl.6: **C12P 19/04**, C12N 1/14, C08B 37/00
// (C12N1/14;, C12R1:645), (C12P19/04;, C12R1:645)

(21) Anmeldenummer: 92103803.0

(22) Anmeldetag: 06.03.1992

(54) **Verfahren zur extrazellulären Herstellung von hochmolekularen Homopolysacchariden und zu ihrer Anwendung, sowie die entsprechenden Pilzstämme**

Process for the extracellular preparation of high molecular weight homopolysaccharides and their use, as well as the corresponding fungus strains

Procédé de préparation extracellulaire d'homo-polysaccharides à haut poids moléculaire et leur application, ainsi que les souches de champignons correspondantes

(84) Benannte Vertragsstaaten:
AT BE DE DK FR GB NL

(30) Priorität: 22.03.1991 DE 4109457

(43) Veröffentlichungstag der Anmeldung:
23.09.1992 Patentblatt 1992/39

(73) Patentinhaber: WINTERSHALL AKTIENGESELLSCHAFT
D-34112 Kassel (DE)

(72) Erfinder:
• Sewe, Kai-Udo, Dr.
W-2847 Barnstorf (DE)
• Wagner, Fritz, Prof. Dr.
W-3300 Braunschweig-Stoeckheim (DE)
• Prokop, Andreas Dr.
W-3300 Braunschweig (DE)
• Olszewski, Egon
W-3300 Braunschweig-Rautheim (DE)

(74) Vertreter: Karau, Wolfgang, Dr. et al
BASF Aktiengesellschaft,
Patentabteilung ZDX - C 6
D-67056 Ludwigshafen (DE)

(56) Entgegenhaltungen:
EP-A- 0 271 907          DE-A- 4 012 238

• JOURNAL OF THE INTERNATIONAL SOCIETY FOR HUMAN AND ANIMAL MYCOLOGY, SABOURAUDIA, Band 15, 1977; D.J. NIEDERPRUEM et al., Seiten 283-295/

## Beschreibung

Die Erfindung betrifft neue Mikroorganismen und ein verbessertes verfahren zur Herstellung von hochmolekularen, ungeladenen Homopolysacchariden (PS) mit Hilfe dieser Mikroorganismen.

Nichtionische Biopolymere haben in der Technik für verschiedene Anwendungsgebiete Verwendung gefunden. Neben dem Einsatz z.B. in der Lebensmittel-, Kosmetik- und pharmazeutischen Industrie ist ein besonders wichtiges Einsatzgebiet die Verwendung von hochmolekularen PS zur sekundären und tertiären Ölförderung. Ein PS-Typ, der die Anforderungen, die an die Polymeren in der tertiären Erdölförderung gestellt werden (vor allem hohe Viskosität, möglichst unabhängig vom Salzgehalt, und Temperaturbeständigkeit, ferner geringe Adsorption an das Gestein sowie hohes Porendurchdringungsvermögen der wäßrigen Lösungen), besonders gut erfüllt, besteht aus einer Hauptkette aus $\beta$-1,3-verknüpften Glucopyranoseeinheiten, von denen jede dritte mit einer weiteren Glucoseeinheit (als Seitenkette) $\beta$-1,6-glycosidisch verknüpft ist. Die mikrobielle Erzeugung derartiger Verbindungen mit Hilfe filamentöser Pilze wird beispielsweise in US 3 301 848 und EP-A-271 907 beschrieben. Die gemäß der US-Patentschrift erhältlichen Produkte haben jedoch relativ geringe Molekulargewichte, und die Kulturen gemäß der Europäischen Offenlegungsschrift sind bei dem Versuch einer kontinuierlichen Verfahrensweise nicht stabil, d.h. die PS-Produktion nimmt zugunsten des Pilzwachstums rasch ab. Nach der Dissertation von S. Münzer (TU Braunschweig 1989) produziert Schizophyllum commune ATCC 38548 in Submerskultur beträchtliche Mengen des oben genannten PS, doch hat sich gezeigt, daß auch dieser Pilzstamm sich bei kontinuierlicher Kultur rasch morphologisch verändert, wobei die PS-Produktion stark nachläßt.

Niederpruem et al. beschreiben in Sabouraudia 15, 283 bis 295 (1977) die mikrobielle Synthese eines $\beta$-1,3-, $\beta$-1,6-Glucans, also eines PS der oben beschriebenen Struktur, durch Kultivierung verschiedener mono- und dikaryotischer Schizophyllum commune-Stämme. Dabei stellte sich heraus, daß alle Dikaryen wesentlich weniger PS produzierten als ihre monokaryotischen Elternmycelien zusammen addiert, und zwar sogar dann, wenn das Dikaryon aus zwei Monokaryen hervorging, die beide für sich sehr gute PS-Produzenten waren. Ähnliche Produktivitäten fanden auch Wang und Miles, Physiol. Plant 17, 573 bis 588 (1964). Auch die höchsten der gefundenen sehr unterschiedlichen Produktivitäten befriedigen jedoch für praktische Zwecke nicht. Eine kontinuierliche Fahrweise ist in beiden Publikationen nicht beschrieben.

Der Erfindung lag die Aufgabe zugrunde, einen Mikroorganismus zu finden, der nicht nur große Mengen des eingangs genannten PS produziert, sondern auch bei kontinuierlicher Kultur stabil bleibt, d.h., weder sich noch seine Produktivität in störender Weise verändert.

Diese Aufgabe wurde durch die Bereitstellung der drei bei der Deutschen Sammlung von Mikroorganismen und Zellkulturen GmbH, Braunschweig, hinterlegten Pilzstämme DSM 6318, 6319 und 6320 sowie durch das Verfahren gemäß Anspruch 2 gelöst. Dazu wurden zunächst die beiden monokaryotischen Elternmycelien des Dikaryons S. commune ATCC 38548 isoliert, und zwar durch Protoplastierung und anschließende Regeneration des dikaryotischen Mycels des genannten Stammes. Dessen Anzucht erfolgte folgendermaßen:

Vorkultur:

Für die Vorkultur wurden 500 ml-Erlenmeyerkolben mit Schikanen verwendet, die mit 100 ml Medium gefüllt waren. Als Inoculum diente eine Stück Mycel einer Schrägagarkultur. Die 5 bis 6 Tage dauernde Inkubation erfolgte bei 27°C auf einer Rotationsschüttelmaschine bei 100 Upm.

Anschließend wurde die erhaltene inhomogene Kulturbrühe 20 bis 30 sec mit einem Ultra-Turrax-Mixstab mit 20.000 Upm unter sterilen Bedingungen homogenisiert (Ultra-Turrax Typ TP 18-10 und Schaft 18 von IKA, Staufen im Breisgau).

Hauptkultur:

Die Hauptkulturen wurden in 1 l-Erlenmeyerkolben ohne Schikanen mit 250 ml Medium durchgeführt. Die Kolben wurden mit 5 % (V/V) der homogenisierten Vorkultur beimpft und bei 27°C auf einer Rotationsschüttelmaschine bei 100 Upm inkubiert.

Die Medien der Vor- und Hauptkultur hatten folgende Zusammensetzung:

| | |
|---|---|
| Glucose . $H_2O$ | 33 g/l |
| techn. Hefeextrakt | 3 g/l |
| $KH_2PO_4$ | 1 g/l |
| $MgSO_4$ . 7 $H_2O$ | 0.5 g/l |

Zur Protoplastierung wurde das Mycel der Hauptkultur nach 48 h Kultivierungsdauer über Nylongaze mit 120 μm Maschenweite filtriert und mehrmals mit Protoplastierungspuffer (0.05 M Maleinsäure-NaOH-Puffer pH 5.8 + 0.5 M MgSO₄ als Osmotikum) gewaschen. Danach wurden 15 g Mycelfeuchtmasse in einem sterilen Becherglas mit 30 ml sterilfiltriertem Protoplastierungspuffer, in dem 300 mg Novozym 234 (Fa. Novo Industrie, Bagsvaerd, Dänemark) gelöst waren, versetzt. Anschließend erfolgte eine Inkubation von 2 bis 3 h auf einer Schüttelmaschine bei 100 Upm, während der die Protoplasten freigesetzt wurden.

Nach Beendigung der Inkubation erfolgte eine Wattefiltration, um die Protoplasten von den übrigen Zellbruchstükken zu trennen. Durch schonende Zentrifugation (40 min bei 1500 g) und anschließende Resuspension in Protoplastierungspuffer konnten die Protoplasten von der lytischen Enzymlösung befreit werden.

Zur Regeneration wurde eine Protoplastensuspension mit 10 ml eines ca. 45°C warmen und flüssigen Regenerationsagarmediums auf eine bestimmte Protoplastenzahl eingestellt. Die im Agarmedium suspendierten Protoplasten wurden dann auf vorbereitete, osmotisch stabilisierte Agarplatten ausgegossen, wobei die Zusammensetzung des Grund- und des Überschichtungsagars identisch war. Die Platten wurden anschließend 5 bis 8 Tage bei 27°C inkubiert und die angewachsenen Kolonien anschließend mikroskopisch untersucht. Der Grund- und der Überschichtungsagar (Regenerations-Agarmedium) hatten folgende Zusammensetzung:

| | |
|---|---|
| Glucose . H₂O | 33 g/l |
| techn. Hefeextrakt | 0.5 g/l |
| Pepton | 2 g/l |
| KH₂PO₄ | 0.46 g/l |
| K₂HPO₄ | 1 g/l |
| MgSO₄ . 7 H₂O | 122 g/l als Osmotikum |
| Agar | 10 g/l |

Nachdem die regenerierten Mycelien einen Durchmesser von ca. 5 mm erreicht hatten, wurden sie mikroskopisch auf Schnallenbildung untersucht. Mycelien, deren Septen keine Schnallen besaßen, wurden als Monokaryen identifiziert, durch Subkultivierung vereinzelt und in Stammhaltung genommen. Auf diese Weise wurden aus drei Protoplastierungsansätzen 13 Monokaryen (= Protoclone) isoliert. Nachfolgend wird immer dann von Protoclonen gesprochen, wenn es sich um Monokaryen handelt, die über die Protoplastierung eines Dikaryons gewonnen wurden.

Da das Ausgangsdikaryon nur zwei genetisch verschiedene Kerne enthielt, konnte eine Zuordnung der isolierten 13 Monokaryen entsprechend ihrem Genom in die Gruppen A (Protoclon A) und B (Protoclon B) anhand von Kreuzungsexperimenten und durch ein Screening der Proteinspektren erzielt werden.

Ein Vergleich von Submerskultivierungen der beiden monokaryotischen Elternmycelien Protoclon A und B mit dem Ausgangsdikaryon S. commune ATCC 38548 ergab, daß, während das Dikaryon innerhalb von 120 h 10 g PS/l produzierte, die beiden Monokaryen im gleichen Zeitraum kaum nachweisbare Mengen desselben synthetisierten. Dieses Ergebnis zeigt, daß der Befund von Niederpruem et al., nach dem die monokaryotischen Elternmycelien mehr PS produzieren als das resultierende Dikaryon, keine Allgemeingültigkeit besitzt.

Die beiden Protoclone wurden mit diversen anderen monokaryotischen S. commune-Stämmen gekreuzt. Aus diesen Kreuzungen gingen Dikaryen hervor, die im Vergleich mit dem Ausgangsdikaryon S. commune ATCC 38548 eine höhere Produktivität hatten, wie an folgenden Beispielen erläutert wird.

Beispiel 1

Kreuzung des Protoclon B-Stammes mit dem monokaryotischen Stamm S. commune ATCC 36481 führt zu S. commune DSM 6320

Die Kreuzung erfolgte in Petrischalen, in deren Mitte eine Stück Mycel beider Pilzstämme in einem Abstand von ca. 1 cm inokuliert wurde. Die Zusammensetzung des Agarmediums entsprach dem Komplexmedium für die Submerskultivierung (s.o.) mit zusätzlich 15 g Agar/l. Die Inkubation erfolgte bei Raumtemperatur und Tageslicht. Sobald die ersten Fruchtkörper sichtbar waren (nach ca. 10 bis 14 Tagen), wurde von dem neuen Dikaryon eine Stammkultur angelegt.

Anschließend wurde das neue Dikaryon DSM 6320 submers in 1 l-Erlenmeyerkolben kultiviert (Kultivierungsbedingungen s.o.). Die Ergebnisse dieser Kultivierung im Vergleich mit den Werten einiger der für diesen Zweck geeignetsten Pilzstämme, darunter des Ausgangsdikaryons S. commune ATCC 38548, sind in Tabelle 1 aufgelistet.

EP 0 504 673 B1

Beispiel 2

Kreuzung des Protoclon A-Stammes mit dem monokaryotischen Stamm S. commune ATCC 36481 → DSM 6319

Die Kreuzung und anschließende Submerskultivierung des Dikaryons S. commune ATCC 36481 x Protoclon A erfolgte gemäß Beispiel 1. Die Ergebnisse der Submerskultivierung sind in Tabelle 1 wiedergegeben.

Beispiel 3

Kreuzung des Protoclon B-Stammes mit dem monokaryotischen Stamm S. commune ATCC 26891 → DSM 6318

Die Kreuzung und anschließende Submerskultivierung des Dikaryons S. commune ATCC 26891 x Protoclon B erfolgte gemäp Beispiel 1. Die Ergebnisse der Submerskultivierung sind in Tabelle 1 wiedergegeben.

Tabelle 1

| Vergleich der Polysaccharidproduktion verschiedener filamentös wachsender Pilze bei einer Submerskultivierung in 1 l-Erlenmeyerkolben auf Komplexmedium. Die Parameter wurden zum Zeitpunkt des Abbruchs der Kultivierung (nach 120 h) ermittelt | | | |
|---|---|---|---|
| Bsp. | MTM (g/l) | PS (g/l) | $Y_{PS/S}$ |
| 1 (S. commune DSM 6320) | 2.5 | 12.0 | 0.4 |
| 2 (S. commune DSM 6319) | 4.5 | 11.0 | 0.36 |
| 3 (S. commune DSM 6318) | 4.1 | 11.5 | 0.38 |
| S. commune ATCC 38548 | 3.9 | 10.0 | 0.35 |
| Pilzstamm DSM 3887[*] | - | 8.7 | 0.29 |
| Pilzstamm DSM 3891[*] | - | 4.0 | 0.16 |
| Pilzstamm DSM 3892[*] | - | 9.0 | 0.27 |

[*] vgl. EP-A-271 907
MTM = Myceltrockenmasse
$Y_{PS/S}$ = Produktertragskoeffizient (g PS/g verbrauchtes Substrat)

Im Gegensatz zu den Ergebnissen von Niederpruem et al., nach denen ausschließlich monokaryotische Mycelien nennenswerte Mengen PS produzierten, haben also die Kreuzungen zwischen den Protoclonen und anderen kompatiblen Monokaryen zu Dikaryen geführt, die sich als exzellente PS-Bildner auszeichnen. Besonders hervorzuheben ist, daß nicht nur alle drei neuen Stämme bei gleicher Kultivierungszeit mehr PS produzieren als einige der geeignetsten bisher bekannten Mikroorganismen, sondern (im Gegensatz zu jenen) in einer kontinuierlichen Kultivierung keine morphologischen Veränderungen und vor allem auch keine Änderung der Produktivität erkennen lassen, wie im Beispiel 4, insbesondere in den Tabellen 2 und 3, gezeigt wird.

Die Gewinnung der eingangs beschriebenen wertvollen PS erfolgt mit Hilfe der erfindungsgemäßen Pilzstämme nach dem üblichen Verfahren, wie es beispielsweise in EP-A-271 907 beschrieben ist. Dazu werden die genannten Pilzstämme bei einer Temperatur von etwa 15 bis 40°C in einem Nährmedium unter Belüftung und Bewegung gezüchtet, vorzugsweise kontinuierlich, und anschließend zweckmäßig für 2 bis 30, vorzugsweise 5 bis 15 min auf 70 bis 90, vorzugsweise 75 bis 85°C erhitzt, sobald die Pilzstämme ausgewachsen sind, danach die Kulturlösung von der Zellmasse abgetrennt und das gebildete PS mit einem Reinheitsgrad im Bereich von 0,03 bis 0,1 Gew.-% Restprotein daraus isoliert, beispielsweise durch Eindampfen oder Sprühtrocknen der Lösung. Das kontinuierliche Verfahren kann zweckmäßig folgendermaßen durchgeführt werden:

Nach einer Inkubationszeit von 50 h in einem 30 l Bioreaktor wird auf kontinuierliche Dosage des Mediums umgestellt. Die Zudosage des Mediums erfolgt mit einer Schlauchpumpe aus einem sterilisierbaren Rührkessel mit einem Arbeitsvolumen von 200 l. Die Füllstandsregelung des Bioreaktors erfolgt durch auf der Flüssigkeitsoberfläche des Bioreaktors befindliche Leitfähigkeitssonden, die bei Kontakt eine Schlauchpumpe ansteuern, um Kultursuspension über einen offenen Ablauf (bedampftes T-Stück) aus dem Reaktor zu führen. Die für den Mikroorganismus spezifischen Grenzen der Durchflußrate D, als Quotient aus zulaufendem Volumenstrom des Mediums und Arbeitsvolumen des Bioreaktors, sind dabei nach unten durch den Erhaltungsstoffwechsel und nach oben durch die maximale Wachstumsgeschwindigkeit festgelegt.

4

Als Nährmedium werden den Pilzstämmen die üblichen, für sie verwertbaren Stickstoff- und Kohlenstoffquellen sowie die zum Wachstum notwendigen anorganischen Ionen zur Verfügung gestellt. Die Konzentration des Nährmediums findet dort ihre obere Grenze, wo das Dreiphasensystem fest/flüssig/Gas zu steif zum Rühren wird. Bei diskontinuierlicher Fahrweise wird die Kultivierung zweckmäßig bei einer Restkonzentration der Kohlenstoffquelle in der Größenordnung von 0,1 Gew.-%, bezogen auf die Kulturbrühe, abgebrochen. Dies gilt insbesondere dann, wenn von dem Pilzstamm 1,3- und/oder 1,6-Glucanase produziert wird, die die gebildeten PS wieder abbauen würde. Diese Glucanasebildung setzt etwa 2 Tage nach Verbrauch der dem Pilz angebotenen Glucose ein.

Die erzeugten nichtionischen PS haben eine Viskosität im Bereich von 50 bis 190 mPa.s bei einer Scherrate $\gamma = 0,3$ s$^{-1}$ bei 40°C, bestimmt mit 0,3 g PS, gelöst in 1000 ml Wasser hoher Salinität (mit bis 100 g/l Alkali-Ionen und bis 30 g/l Erdalkali-Ionen). Diese Viskosität bleibt bei einer Temperatur von 60°C an der Luft 1 Jahr und länger praktisch konstant.

Unter Sauerstoffausschluß gilt dies sogar für Temperaturen bis 90°C. Die Viskosität der erfindungsgemäß erzeugten nichtionischen PS wird bei einer Konzentration von 0,1 bis 1 g/l im Temperaturbereich von etwa 20 bis 60°C und und im Scherbereich von etwa $\gamma = 3,0$ s$^{-1}$ und größer nur wenig beeinflußt, so daß sie für praktische Zwecke als konstant angesehen werden kann. Auch nach einstündigem Erhitzen einer 0,3 g/l enthaltenden Lösung der erfindungsgemäß erhältlichen PS im Autoklaven bei neutralem pH-Wert auf 120°C bei 1 bar Überdruck ist die Viskosität nach Abkühlen auf 20°C und Ergänzen des Verdunstungswassers im Scherbereich von $\gamma = 0,1$ bis 300 s$^{-1}$ praktisch nicht beeinflußt.

Die Pilzstämme können für den Einsatz an einer Trägersubstanz, vorzugsweise einem Polyurethanschaum, in üblicher Weise immobilisiert werden, und mit diesem Immobilisat kann die Bildung der PS semikontinuierlich oder vollkontinuierlich durchgeführt werden.

Die erfindungsgemäß erhältlichen PS eignen sich hervorragend zum Einsatz als Dispergier- oder Emulgiermittel (allein oder in Kombination mit anionischen und/oder nichtionischen Tensiden), als strukturviskose Verdickungsmittel, als Geldbildner, als Wasserrückhaltemittel sowie für die Verbesserung der Haftung von Stoffen an festen Oberflächen. Bei der Verwendung als Verdickungsmittel ist insbesondere an den Einsatz bei Bohrspülungen, bei sekundärem und tertiärem Fluten von Erdöllagerstätten und generell zur Herabsetzung von Reibungswiderständen in strömenden, insbesondere in turbulenten wäßrigen Flüssigkeiten zwecks Herabsetzung des Druckverlustes gedacht. Als Dispergiermittel kommen beispielsweise Anwendungen bei landwirtschaftlichen Chemikalien wie Fungiziden, Pestiziden sowie im Nahrungsmittel- und Futtermittelbereich in Betracht.

Die Filtrationseigenschaften der erfindungsgemäß erzeugten PS als Ausdruck der Injizierbarkeit in poröse Medien wie Erdölträgergestein sind hervorragend: Sie liegen bei einer Probe von 0,3 g PS/l Wasser mit hoher Salinität mit bis 100 g/l Alkali-Ionen und bis 30 g/l Erdalkali-Ionen bei einer Druckfiltration mit einem bar Überdruck über ein 3 μm Membranfilter mit 200 ml Lösung 5 sec. geschert im Bereich von 0,5 bis 3,0 min. und ungeschert im Bereich von 0,8 bis 180min., oder über ein 1,2 μm Membranfilter 5 sec. geschert im Bereich von 1,6 bis 5 min. und ungeschert im Bereich von 4,0 bis 300 min..

Beispiel 4

Kultivierungsbedingungen für die drei neuen Schizophyllum commune-Stämme DSM 6318 bis 6320:

Die Vorkultivierung und Batch-Anzucht erfolgte in folgendem Medium:

| Glucose: | 30 g l$^{-1}$ |
|---|---|
| techn. Hefeextrakt: | 3 g l$^{-1}$ |
| MgSO$_4$: | 0,5 g l$^{-1}$ |
| KH$_2$PO$_4$: | 1 g l$^{-1}$ |

Nach einer Kultivierungsdauer von ca. 50 h in einem 30 l Bioreaktor, ausgerüstet mit 3 Schrägblattrührern mit einem Durchmesserverhältnis von D = 0,64, wurde wie oben beschrieben auf kontinuierliche Betriebsführung umgestellt.

Da die Produktivität des Mikroorganismus von der Homogenität der Kulturbrühe abhängt, wird bei überschreiten des mittleren Durchmessers der Zellagglomerate von ca. 1 mm die Kulturbrühe über eine Zahnradpumpe (Typ V 150.12, Verder) rezyklisiert. Eine hinreichende Homogenität der Kulturbrühe während der kontinuierlichen Fermentation ergibt sich bei 4 Zyklen pro Volumenaustausch und pro gebildetem Gramm Biomasse pro Liter. Diese Scherzyklenzahl wurde empirisch ermittelt und stellt einen Kompromiß aus höherer Zelloberfläche, vermehrter Glucanablösung von der Zellwand und geringer Zellschädigung dar.

Eine pH-Regelung fand nicht statt. Die kontinuierlich anfallende Kulturbrühe wird, wie in EP-A-271 907 beschrieben, diskontinuierlich aufgearbeitet.

Konzentration der Edukte im Zulauf bei kontinuierlicher Prozeßführung:

| Glucose: | 0,80 g l⁻¹ h⁻¹ |
|---|---|
| techn. Hefeextrakt: | 0,08 g l⁻¹ h⁻¹ |
| $MgSO_4$: | 0,01 g l⁻¹ h⁻¹ |
| $KH_2PO_4$: | 0,02 g l⁻¹ h⁻¹ |

Kultivierungsbedingungen

Die folgenden Tabellen zeigen die stationären Biomasse- und PS-Konzentrationen für S. commune DSM 6320 während der kontinuierlichen Fermentation in Abhängigkeit der Kultivierungsdauer unter folgenden Prozeßparametern (die auch für die Vorkultivierung und Batch-Anzucht gelten):

| Temperatur: | $T = 27°C$ |
|---|---|
| Begasungsrate: | 0,08 vvm (Volumen/Volumen·Minute) |
| Rührgeschwindigkeit: | 150 min⁻¹ |
| Durchflußrate: | $D = 0.072 h^{-1}$ |

Tabelle 2

| Zeit [Tage] | $X^1$ [g l⁻¹] | PS [g l⁻¹] |
|---|---|---|
| 2 | 0.97 | 5.4 |
| 8 | 1.10 | 5.4 |
| 14 | 1.05 | 5.5 |
| 18 | 1.02 | 5.4 |
| 31 | 0.98 | 5.4 |

[1] X = Zelltrockenmasse

Tabelle 3

| Bei einer Durchflußrate von $D = 0.04 h^{-1}$ und einer Rührgeschwindigkeit von $N = 100 min^{-1}$ ergeben sich unter sonst gleichen Bedingungen folgende Analysenwerte: | | |
|---|---|---|
| Zeit [Tage] | X [g l⁻¹] | PS [g l⁻¹] |
| 5 | 0.41 | 4.1 |
| 11 | 0.39 | 4.0 |
| 23 | 0.38 | 3.9 |
| 43 | 0.41 | 4.2 |
| 50 | 0.39 | 4.2 |
| 68 | 0.42 | 4.1 |
| 75 | 0.41 | 4.1 |

Die unterschiedlichen Durchflußraten spielen hier keine Rolle, da das System in beiden Fällen stabil (im Gleichgewicht) ist. Wohl aber wirkt sich die unterschiedliche Belüftungsrate (Rührgeschwindigkeit) aus.

Physikalische Kenndaten der von S. commune DSM 6318, 6319 und 6320 unter den angegebenen Kultivierungsbedingungen produzierten PS:

DSM 6318 MG = 14,8 · 106 g/mol

DSM 6319 MG = 12,2 · 106 g/mol

DSM 6320 MG = 17,8 · 106 g/mol

Die Molekulargewichte MG wurden über den Staudinger Index wie folgt bestimmt:

Das mittlere Molekulargewicht MG der PS steht über die Mark-Houwink Gleichung mit dem leicht meßbaren Staudinger Index $[\eta]$ in folgender Beziehung:

$$[\eta] = K \cdot MG^a \qquad K = 4{,}45 \ 10^{-7}$$

$$MG = ([\eta]/K)^{1/a} \qquad a = 1{,}49$$

Temperaturbeständigkeit:

Bei einer Produktkonzentration von 0.3 g l$^{-1}$ wurde bei einer Scherrate von 0.3 s$^{-1}$ eine Viskosität von 152 mPas bei einer Temperatur von 40°C gefunden. Zehntägiges Aufbewahren einer Produktprobe an der Luft bei einer Temperatur von 80°C zeigte keine Änderung der Viskosität.

pH-Stabilität:

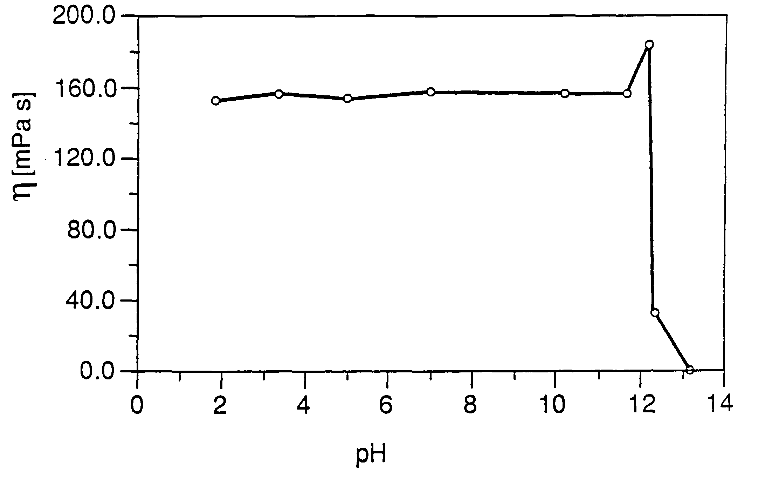

Die Viskosität, gemessen unter den oben aufgeführten Bedingungen, ist, wie Figur 1 zeigt, in einem weiten Bereich unabhängig vom pH-Wert. Erst bei Überschreiten des pH-Wertes von 12.2 kommt es, bedingt durch eine Konformationsänderung, nach kurzem Anstieg zu einem drastischen Abfall der Viskosität.

Die von S. commune DSM 6318 und 6319 produzierten PS haben etwa die gleichen physikalischen Kenndaten. Die Molekulargewichte, gemessen nach der oben genannten Methode, liegen im Bereich von 5 bis 25, vorzugsweise 12 bis 25 Millionen. DSM 6320 wird besonders bevorzugt, weil dieser Stamm die höchste Produktivität zeigt.

Die Pilzstämme sind "ausgewachsen", wenn sie die ihnen zur Verfügung stehende Nährlösung ganz oder weitgehend verbraucht haben. Auf das Erhitzen des Kulturmediums nach dem Ausreifen der Pilzstämme kann man im Prinzip verzichten, wenn man sicher ist, daß die PS-Lösung nach dem Abtrennen der Biomasse keinen einzigen Bioorganismus mehr enthält.

Die PS der drei neuen Pilzstämme sind in der Struktur gleich und im Molekulargewicht und damit in allen Eigenschaften den besten bisher bekannten gleichwertig. Die Erfindung liegt in der Schaffung der neuen Schizophyllum commune-Stämme DSM 6318 bis 6320, die diese hochwertigen PS in relativ großen Mengen und in kontinuierlichem Verfahren produzieren, was bisher nicht möglich war.

**Patentansprüche**

1. Die Pilzstämme DSM 6318, DSM 6319 und DSM 6320.

2. Verfahren zur extrazellulären Herstellung von Homopolysacchariden vom Molekulargewicht 5 bis 25 Millionen mit in der Hauptkette ausschließlich β-1,3-D-Glucopyranose-Einheiten, von denen jede dritte mit einer weiteren Glucose-Einheit β-1,6-glycosidisch verknüpft ist, dadurch gekennzeichnet, daß Mikroorganismen in Form von mindestens einem der Pilzstämme DSM 6318, DSM 6319 und DSM 6320 in einem Nährmedium unter Belüftung und Bewegung bei einer Temperatur im Bereich von 15 bis 40°C gezüchtet werden, danach die Kulturlösung von der Zellmasse abgetrennt und das gebildete wasserlösliche Homopolysaccharid daraus in üblicher Weise isoliert wird.

3. Verfahren nach Anspruch 2, dadurch gekennzeichnet, daß als Nährmedium eine wäßrige Lösung von Stärke, Hemizellulose, Glucose oder Saccharose eingesetzt wird in einer solchen Konzentration, daß die Kultursuspension in dem 3-Phasen-System fest/flüssig/Gas in jedem Reifestadium noch mischbar ist.

4. Verfahren nach Anspruch 2 oder 3, dadurch gekennzeichnet, daß bei Pilzstämmen, deren β-1,3- oder β-1,6-Glucanase oder beide positiv sind, bei einer Restkonzentration an Kohlenstoffsubstrat von etwa 0,1 Gew.-% die Kultivierung abgebrochen wird.

5. Verfahren nach einem der Ansprüche 2 bis 4, dadurch gekennzeichnet, daß es kontinuierlich oder semikontinuierlich durchgeführt wird.

6. Verfahren nach Anspruch 5, dadurch gekennzeichnet, daß die Pilzstämme vor dem Einsatz an einer Trägersubstanz nach üblichen Methoden immobilisiert werden.

7. Verwendung der nach einem der Ansprüche 2 bis 6 erhaltenen Homopolysaccharide als Dispergier- und Emulgiermittel, als Wasserrückhaltemittel, zur Verbesserung der Haftung von Stoffen an festen Oberflächen und als Verdickungsmittel.

8. Verwendung der nach einem der Ansprüche 2 bis 6 erhaltenen Homopolysaccharide als Hilfsmittel bei der tertiären Ölförderung.

**Claims**

1. The fungal strains DSM 6318, DSM 6319 and DSM 6320.

2. A process for the extracellular preparation of homopolysaccharides of molecular weight 5 - 25 million with exclusively β-1,3-D-glucopyranose units in the main chain, each third of which is β-1,6-glycosidically linked to another glucose unit, which comprises culturing microorganisms in the form of at least one of the fungal strains DSM 6318, DSM 6319 and DSM 6320 in a nutrient medium with aeration and agitation at from 15 to 40°C, then separating the culture solution from the biomass and isolating the resulting water-soluble homopolysaccharide therefrom in a conventional manner.

3. A process as claimed in claim 2, wherein an aqueous solution of starch, hemicellulose, glucose or sucrose is employed as nutrient medium in a concentration such that the culture suspension in the solid/liquid/gas 3-phase system can be mixed in every stage of growth.

4. A process as claimed in claim 2 or 3, wherein the cultivation of fungal strains whose β-1,3- or β-1,6-glucanase or both are positive is stopped at a residual concentration of carbon substrate of about 0.1% by weight.

5. A process as claimed in any of claims 2 to 4, which is carried out continuously or semicontinuously.

6. A process as claimed in claim 5, wherein the fungal strains are immobilized by conventional methods on a carrier substance before use.

7. The use of the homopolysaccharides obtained as claimed in any of claims 2 to 6 as dispersants and emulsifiers, as humectants, to improve the adhesion of materials to solid surfaces and as thickening agents.

8. The use of the homopolysaccharides obtained as claimed in any of claims 2 to 6 as aids in tertiary oil extraction.


**Revendications**

1. Les souches de champignons DSM 6318, DSM 6319 et DSM 6320.

2. Procédé de préparation extra-cellulaire d'homopolysaccharides d'un poids moléculaire de 5 à 25 millions avec exclusivement des unités β-1,3-D-glucoparynose dans la chaîne principale dont chaque troisième unité est combinée à une autre unité glucose de type β-1,6-glycosidique, caractérisé en ce que des micro-organismes se présentant sous la forme d'au moins l'une des souches de champignons DSM 6318, DSM 6319 et DSM 6320 sont élevés dans un milieu nutritif avec aération et déplacement, à une température située dans la plage allant de 15 à 40°C, puis la solution de culture est séparée de la masse cellulaire et l'homopolysaccharide soluble dans l'eau constituée en est isolé, de la manière usuelle.

3. Procédé selon la revendication 2, caractérisé en ce que l'on utilise, comme milieu nutritif, une solution aqueuse d'amidon, d'hémicellulose, de glucose ou de saccharose sous une concentration telle que la suspension de culture est susceptible d'être mélangée encore à toutes les étapes de maturité dans le système à trois phases solide/liquide/gazeux.

4. Procédé selon la revendication 2 ou 3, caractérisé en ce que pour les souches de champignons dont le β-1,3 ou le β-1,6 glucanase ou les deux sont positifs, la culture est arrêtée lorsque l'on atteint une concentration résiduelle d'à peu près 0,1 % en poids en substrat de carbone.

5. Procédé selon l'une des revendications 2 à 4, caractérisé en ce qu'il est conduit de façon continue ou semi-continue.

6. Procédé selon la revendication 5, caractérisé en ce que, avant Utilisation, les souches sont immobilisées sur une substance support, selon les méthodes usuelles.

7. Utilisation des homopolysaccharides obtenus selon l'une des revendications 2 à 6, comme dispersant ou émulsifiant, comme moyen de rétention d'eau, pour améliorer l'adhérence de substances sur des surfaces solides et comme épaississant.

8. Utilisation des homopolysaccharides obtenus selon l'une des revendications 2 à 6, comme auxiliaire lors de l'extraction d'huile tertiaire.